# EUROPEAN PATENT APPLICATION

(11) **EP 2 198 907 A1**
(43) Date of publication of application: **23.06.2010**
(21) Application number: 08022091.6
(22) Date of filing: 19.12.2008
(51) Int. Cl.: A61M 15/00

(54) **Inhaler**

(71) Applicant: Boehringer Ingelheim International GmbH, 55216 Ingelheim am Rhein (DE); Vectura Delivery Devices Limited, Chippenham, Wiltshire SN14 6FH (GB)
(72) Inventor: The designation of the inventor has not yet been filed
(74) Representative: Grey, Ian Michael

(57) **Abstract**

An inhaler (1) is proposed for delivering a preferably powdered formulation from a blister strip having a plurality of blister pockets (3). Each blister pockets is pierced such that an inlet opening and an outlet opening is fanned in a lid of the respective blister pocket. A sealing (21) is provided between the two openings (18,19) and/or only around the outlet opening (19) in order to avoid a bypass gas/air stream. Alternatively, a blister strip (7) is proposed which comprises sealings on lids of its blister pockets (3).

## Description

The present invention relates to an inhaler according to the preamble of claim 1 and to a carrier according to the preamble of claim 13.

The present invention relates to passive inhalers, i.e. inhalers where the patient or user breathes in to generate an air stream, which entrains the inhalation formulation and forms the desired aerosol.

The present invention relates to an inhaler for delivery of a powder-form inhalation formulation from a blister strip with a plurality of blister pockets (also called blisters) containing the inhalation formulation in doses,

GB 2 247 042 A discloses an inhaler with a rolled-up blister strip. The inhaler comprises a manually operated, pivotable actuator, which operates a conveyor for stepwise moving the blister strip. The actuator supports a piercer and an associated mouthpiece, By pivoting the actuator, the blister strip is moved forward and blister pockets of the blister strip can be pierced one after the other. When a patient breathes in an air stream passes through the previously pierced blister pocket, with the result that the inhalation formulation in the blister pocket mixes with the air and is discharged to the patient.

The design of a piercing member for puncturing the blister pockets is important in order to achieve good discharge characteristics. Multiple piercing member designs are known. However, studies have shown that the present piercer designs do not result in optimized discharge characteristics and/or are difficult to produce and/or have a complicated design.

CA 2 555 600 A1 discloses in Fig. 17 to 19 an inhaler with a removal device in the form of a piercer having multiple piercing elements to form inlet openings and an outlet opening in a cover of a receptacle for discharging a powdered medicament contained in the receptacle by means of an air stream flowing into the receptacle through the inlet openings and out of the receptacle through the outlet opening. The piercer comprises a gasket surrounding the piercing elements and contacting the pierced cover so that the inlet and outlet openings are encircled by this common gasket.

It has been found that an undesired bypass air stream can flow from the inlet openings to the outlet opening on the outside of the cover, and, thus, bypass the receptacle. Such a bypass air stream may negatively influence the discharge characteristics of the inhaler.

Object of the present invention is to provide an inhaler and a carrier for an inhaler with improved discharge characteristics.

The above object is achieved by an inhaler according to claim 1 or by a carrier for an inhaler according to claim 13. Advantageous embodiment are subject of the subclaims.

According to the present invention, a sealing, in particular a gasket, is provided or formed between the inlet opening and the outlet opening and/or only around the outlet opening to prevent, minimize or restrict a bypass gas / air stream flowing from the inlet opening to the outlet opening on the outside of the cover or lid of the opened blister pocket. In particular, the bypass gas / air stream between the lid and an associated removal device for opening the blister pocket can be minimized or even prevented. Thus, improved discharge characteristics of the inhaler can be achieved. In particular, discharge characteristics can be achieved that are at least highly repeatable or similar from one blister pocket to the next one.

According to another aspect of the present invention, a carrier, in particular a blister strip, is proposed which comprises scalings in order to prevent, minimize or restrict bypass gas / air streams when discharging receptacles of the carrier individually by a gas / air stream.

Further aspects, features, properties and advantages of the present invention are described in the claims and the subsequent description of a preferred embodiment, with reference to the drawing. There are shown in:
- Fig. 1: a schematic sectional view of an inhaler without mouthpiece cover;
- Fig. 2: a schematic sectional representation of the inhaler with closed mouthpiece cover;
- Fig. 3: a schematic sectional view of the inhaler in the area of a mouthpiece with a removal device;
- Fig. 4: a partial view of a carrier in the form of a blister strip;
- Fig. 5: a bottom view of the removal device according to fig. 3;
- Fig. 6: a schematical sectional view of another removal device;
- Fig. 7: a perspective view of a further removal device; and
- Fig. 8: a schematic view of a blister strip.

In the Figures, same reference numbers are used for identical or similar parts, even if a repeated description is omitted. In particular identical or corresponding advantages and properties then also result or may be achieved.

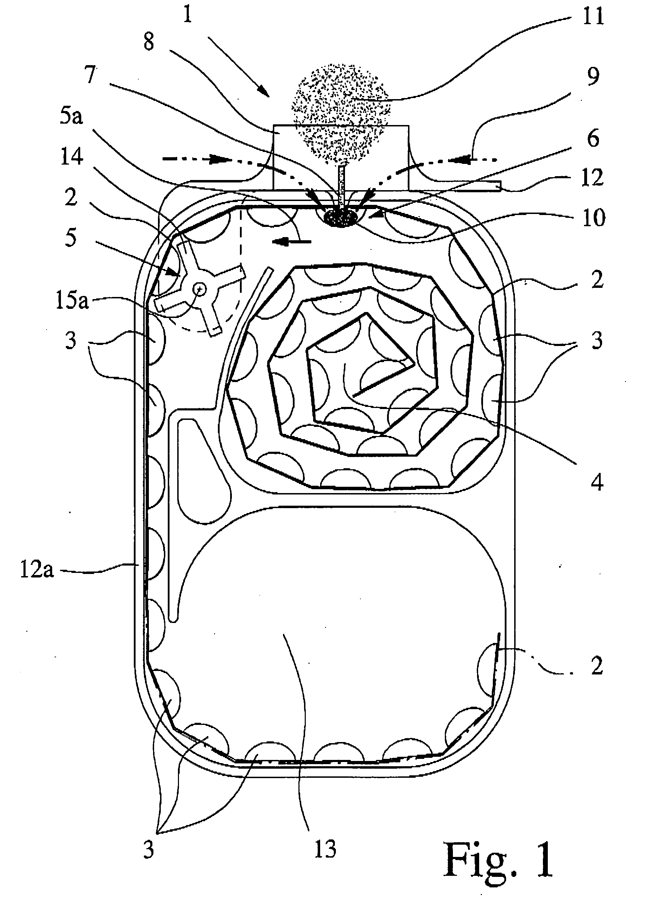

Fig. 1 shows in a very schematic sectional representation an inhaler 1,

The inhaler 1 serves to deliver a powdered inhalation formulation from a preferably band-shaped carrier, such as a blister strip 2. The blister strip 2 is finite, not forming an endless or closed loop. It has a large number of blister pockets 3 respectively containing directly a dose of the loose inhalation formulation, in particular powder 10. Thus, the formulation is pre-metered.

The inhaler 1 has a reservoir 4 for the still unused blister strip 2 with closed (sealed) receptacles, here blister pockets 3. The blister strip 2 is preferably rolled up or wound up in the reservoir 4. In the representation example the reservoir 4 is formed such that the blister strip 2 can be moved outwards or pulled out of the reservoir 4 as easily as possible.

In the representation example the blister strip 2 is directly received in the reservoir 4. However, instead of this a cassette, a container, a drum or suchlike can also be fitted or inserted with the blister strip 2 into the inhaler 1 or the reservoir 4.

The inhaler 1 has preferably a conveyor 5 for stepwise onward movement of the blister strip 2 in direction of arrow 5a by one blister pocket 3, in order to feed the blister pockets 3 successively to an opening and/or removal position 6 where the respective blister pocket 3 is opened and can be emptied.

The blister pockets 3 can be opened respectively preferably by means of a removal device or piercing member 7, which punctures or cuts open a lid of the respectively aligned blister pocket 3 in position 6. The piercing member 7 is hollow and/or in fluid connection with an adjacent mouthpiece 8 of the inhaler 1. Preferably, the inhaler 1 is constructed such that the receptacles (blister pockets 3) can be individually opened from the outside one after another.

During or for inhalation a patient or user, not represented, places the mouthpiece 8 in his mouth and breathes in. An air stream 9 of ambient air is sucked in and passed through the opened blister pocket 3 such that the loose powder 10 (forming the inhalation formulation and being schematically shown in Fig. 1 only in the actually opened blister pocket 3 below mouthpiece 8) is dispensed with the sucked-in ambient air as an aerosol cloud 11 via the mouthpiece 8. This situation is schematically represented in Fig. 1. Thus, the respectively opened blister pocket 3, into which the piercing member 7 partly extends, is emptied.

The inhaler 1 has a preferably manually actuatable, lever-like actuator 12 being pivotally mounted to a housing 12a of the inhaler 1. The piercing member 7 and the mouthpiece 8 are attached to and supported by the actuator 12.

The actuator 12 is operable (pivotable) to cause the piercing member 7 to puncture the lid of the respectively aligned blister pocket 3 in position 6 below the mouthpiece 8.

When the actuator 12 swivels from the position shown in Fig. 1 (here anticlockwise) to a partially opened position, the piercing member 7 is withdrawn from the last-pierced blister pocket 3.

Then, the blister strip 2 is moved forward by one blister pocket 3, so that the next blister pocket 3 is moved in position 6. This will be explained in more detail later.

When the actuator 12 swivels back into the position shown in Fig. 1, i.e. is manually moved back, the next aligned blister pocket 3 of the blister strip 2 is punctured by the piercing member 7 and thereby opened. Then, the next inhalation can take place, i.e. the inhaler 1 is activated.

The inhaler 1 has preferably a receiving space or apparatus 13 to receive or store the used part of the blister strip 2. The receiving space or apparatus 13 is formed such that the used part can be wound up. Fig. 1 shows a situation with essentially filled reservoir 4 and still essentially empty receiving space 13.

The conveyor 5 comprises preferably a conveying wheel 14, which can engage between the blister pockets 3 and thus convey the blister strip 2 in form-locking or form-fit manner. This allows very secure or precise moving or indexing of the blister strip 2 as desired and/or necessary.

The conveyor 5 or its conveying wheel 14 is arranged preferably between the reservoir 4 and the receiving apparatus 13, in particular between the removal position 6 and the receiving apparatus 13, thus after the emptying of the blister pockets 3,

The pivot axis of the actuator or lever 12 is coaxial with the rotation axis of the conveying wheel 14. In particular, the actuator or lever 12 may be supported by an axle of the conveying wheel 14.

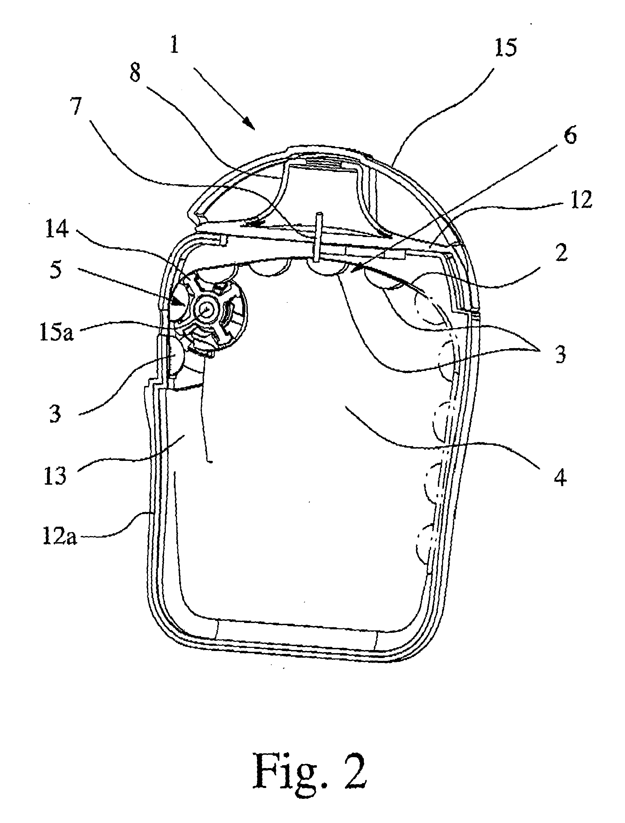

The inhaler 1 comprises a mouthpiece cover 15. The mouthpiece cover 15 is not shown in Fig. 1, which explains only the basic principle of the inhaler 1, but in Fig. 2, which shows a more realistic, but still very schematic sectional view of the inhaler 1. Fig. 2 shows the inhaler 1 with closed mouthpiece cover 15, wherein the blister strip 2 has been partly omitted for illustration purposes.

The mouthpiece cover 15 is pivotable around a cover axis 15a, which is indicated in Fig. 1 and 2 and extends perpendicular to the drawing plane in the present representation.

The pivot axis of the actuator 12 extends preferably coaxial to or with the cover axis 15a. The rotation axis of the conveying wheel 14 extends preferably coaxial to the cover axis 15a and to the pivot axis of the actuator 12.

The conveyor 5 or its conveying wheel 14 is preferably driven by the mouthpiece cover 15, namely by the pivotal movement of the mouthpiece cover 15. In particular, the blister strip 2 is moved forward, when the mouthpiece cover 15 is opened. Preferably, only part of the opening movement of the mouthpiece cover 15 actuates or operates the conveyor 5 or its conveying wheel 14 to move the blister strip 2 forward.

When the mouthpiece cover 15 is opened starting with the completely closed position shown in Fig. 2, in a first phase of the opening movement, for example up to a first angle of about 10, 20 or 30 degrees, the blister strip 2 is not moved due to a respective freewheel (not shown) between the mouthpiece cover 15 and the conveying wheel 14.

First of all, the actuator 12 has to be moved or opened in order to withdraw the piercing member 7 from the previously pierced and usually already emptied blister pocket 3. This opening movement of the actuator 12 can be performed manually. However, the actuator 12 preferably opens automatically when opening the mouthpiece cover 15, In particular, the mouthpiece cover 15 can be opened up to the first angle. When the mouthpiece cover 15 reaches this angle, e.g. about 20 degrees, the actuator 12 flips automatically open into its opened position, in particular due to a biasing or spring means (not shown) or the like. However, it also possible that the actuator 12 moves jointly with the mouthpiece cover 15 in the first phase of the opening movement (e.g. due to a ratchet mechanism, spring or the like) until the actuator 12 reaches its opened position or the first angle,

The opened position of the actuator 12 is preferably set such that the piercing member 7 is not exposed to the exterior and/or that the inhaler 1 is not completely opened in order to avoid or at least minimize any potential external influences.

In order to limit the open position of the actuator 12, the opening or pivot range of the actuator 12 is smaller than the one of the mouthpiece cover 15 and/or is restricted to preferably at most 20 degrees, in particular to about 10 degrees or less.

However, it is also possible that the actuator 12 is not limited in its opening position, but can open or pivot as far as the mouthpiece cover 15, in particular jointly with the mouthpiece cover 15.

During the further opening (second phase) of the mouthpiece cover 15, the conveyor 5 or its conveying wheel 14 is actuated to move or index the blister strip 2 by one blister pocket 3 onward to the next blister pocket 3 that shall be emptied. This blister movement happens preferably up to the complete opening of the mouthpiece cover 15.

Only when the mouthpiece cover 15 is opened completely, i.e. reaches its end position, the movement of the blister strip 2 may be set or fixed by a respective mechanism (not shown) and/or decoupled from the mouthpiece cover movement to keep the next blister pocket 3 in position 6 for puncturing. However, if the mouthpiece cover 15 is not fully opened and closed again, then, the blister strip 2 is moved backward. This facilitates operation of the inhaler 1 and, in particular, prohibits that incomplete or unintended operation of the mouthpiece cover 15 results in an undesired movement of the blister strip 2 and eventually in an undesired opening of the next blister pocket 3.

Preferably, a lock (not shown) is provided so that the opened actuator 12 can be closed again only if the mouthpiece cover 15 has been fully opened or pivoted back to the first angle or the last-pierced blister pocket 3 has been moved back into position 6. Thus, the piercing member 7 cannot be pushed against an area of the blister strip 2 without or beside a blister pocket 3.
When the mouthpiece cover 15 has been fully opened and the next blister pocket 3 has been moved in position 6, the actuator 12 can be pivoted back, i.e. closed, in order to pierce the already aligned, still closed blister pocket 3. Then, the inhaler 1 is ready for inhalation, i.e. activated as already described.

After inhalation, the inhaler 1 can be closed by pivoting back the mouthpiece cover 15 into its closed position.

In order to operate the conveyor 5 or its conveying wheel 14 by movement of the mouthpiece cover 15 as described above or in any other suitable manner, the mouthpiece cover 15 is coupled with the conveyor 5, in particular the conveying wheel 14, via the already mentioned freewheel, a suitable transmission, a slipping clutch and/or any other suitable coupling or the like. Preferably, the freewheel, transmission, coupling or the like is integrated into or located adj acent to the conveying wheel 14 or a respective axle.

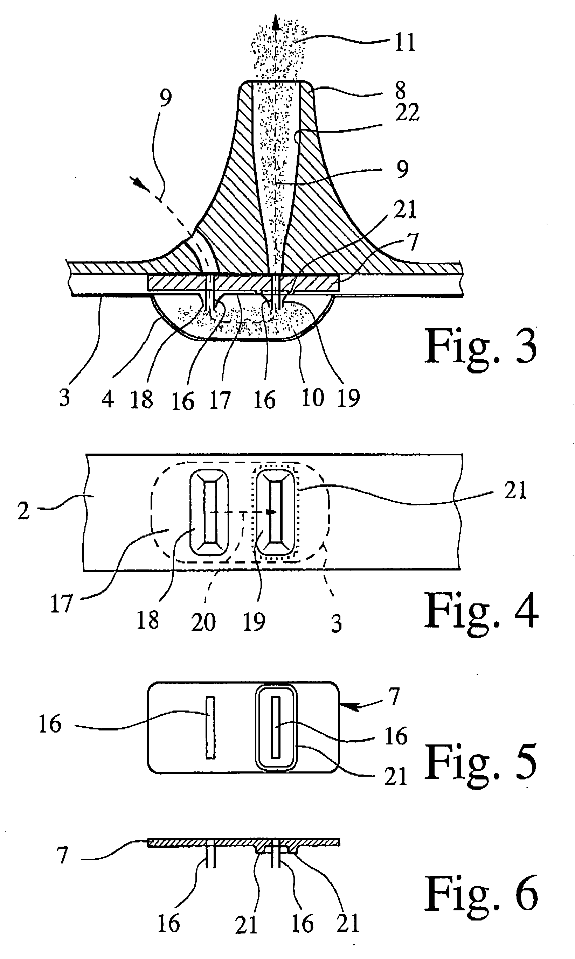

Generally, the removal device 6 is/or moved or displaced transversally, preferably essentially perpendicular, to the direction of conveying or onward movement 5a or in the radial direction, in order to open and fluidically connect the next respective receptacle or blister pocket 3 as shown in Fig. 3. In this removal position 6, the opened receptacle abuts or contacts the removal device 7, in particular a lid 17 of the receptacle / blister pocket 3 contacts a corresponding contact area formed by the removal device 7 or any other component of the inhaler 1.

The removal device 7 comprises preferably at last two opening or piercing elements 16 for puncturig or opening the respective receptacle blister pocket 3.

When the respective receptacle is opened, an inlet opening 18 is formed through which the gas or air stream 9 can enter into the opened blister pocket 3, and an outlet opening 19 is formed through which the gas or air stream 9 can flow again out of the opened blister pocket 3.

Fig. 4 shows in a schematic, partial view the carrier / blister strip 3, in particular an opened blister pocket 3 with the inlet opening 18 and outlet opening 19 in the lid 17. It has to be noted that the form of the inlet opening 18 and/or outlet opening 19 may be much more irregular or different or similar. In particular, the inlet opening 18 can be larger than the outlet opening 19 or vice versa. The inlet opening 18 and outlet opening 19 are preferably spaced from each other.

It has been found that gas or air may bypass the blister pocket 3 in that the gas or air flows on the outside of the lid 17 - in particular, between the lid 17 and the preferably essentially flat or plate-like surface or contact surface of the removal device 7 - directly from the inlet opening 18 to the outlet opening 19 as schematically shown in Fig. 4 by arrow 20 representing a bypass gas/air stream. This undesired bypass gas/air stream 20 can in particular occur when the blister pocket 3 or its lid 17 does not plainly or tightly contact the contact surface of the removal device 7 or any other adjacent component of the inhaler 1. This bypass gas/air stream 20 may negatively influence discharge characteristics of the inhaler 1. Therefore, it is desired to prevent or at least minimize this potential bypass gas/air stream 20 between the inlet opening 18 and the outlet opening 19 and/or between two opening elements 16 of the removal device 7. This is achieved according to the present invention in that a sealing 21 is provided or formed between the inlet opening 18 and the outlet opening 19 and/or only around the outlet opening 19. Fig. 4 shows the preferred line (dotted) or area where the sealing 21 contacts the lid 17.

In particular, the sealing 21 is arranged outside the receptacles / blister pockets 3 / lid 17 and/or carrier / blister strip 3. In particular, the sealing 21 contacts the opened lid 17 from the outside and/or is pressed or biased against the lid 17 in the removal position of the opened blister pocket 4, i.e. when the blister pocket 3 is fluidically connected so that its dose of formulation can be discharged as shown in particular in Fig. 1 and 3.

In a preferred embodiment, the carrier and the removal device 7 may even be pressed together in the removal position 6, in particular the lid 17 onto the removal device 7 or vice versa, e.g. due to a spring force, elastic forces of the carrier and/or other biasing forces in order to tightly press the sealing 21 onto the lid 17.

It has to be noted that the sealing 21 can be used alternatively to restrict or define the bypass gas/air stream 20 so that well-defined or reliable or repeatable discharge characteristics of the inhaler 1 can be achieved as well. In particular, if a certain or predetermined bypass gas/air stream 20 is allowed by respective design of the sealing 21, other bypass paths usually used to mix the aerosol cloud 11 in an outlet path 22 with additional gas or air could be avoided. Additionally or alternatively, the design of the sealing 21 may also define the flow resistance of the inhaler 1 when inhaling.

The sealing 21 may be a gasket and/or at least partially elastically deformable. In particular, the sealing 21 may comprise or be formed by an elastic and/or flexible element as schematically shown in Fig. 3 and 5. The sealing 21 preferably forms an elastic and/or flexible ridge, rib, lip or the like.

Fig. 5 shows in a bottom view the removal device 7 with preferably two opening elements 16 and the sealing 21.

Preferably, the sealing 21 is arranged, extends and/or forms a barrier between the inlet and outlet openings 18, 19 and/or between the opening elements 16. This may already reduce or restrict the bypass gas/air stream 20,

The sealing 21 forms or comprises preferably a ring or loop arrangement, in particular a closed loop, and/or encompasses only the outlet opening 19 and/or only the opening element 16 of the removal device 7 forming the outlet opening 19 as shown in Fig. 3 to 5,

Preferably, the sealing 21 reduces the contact area between the lid 17 and the contact surface. In particular, the resulting contact surface is essentially like a line as schematically shown in Fig. 3 to 5 so that high contact pressures and, thus, very good sealing effects can be achieved.

Fig. 6 shows in a schematic side view another embodiment of the removal device 7 and/or sealing 21. Here, the sealing 21 forms a preferably inelastic, flat and/or rib-like a protrusion, barrier or elevated plane area encompassing, separating and/or supporting preferably only the opening element 16 of the removal device 7 forming the outlet opening 19. Even with this design, the contact area mentioned above is reduced and thus, an improved sealing effect achieved.

Preferably, the edges of the sealing 21 are relatively sharp in order improve the sealing effect and/or to achieve a line contact.

It has to be noted that the sealing 21 can also be rigid or semi-rigid, in particular in the embodiment shown in Fig. 6.

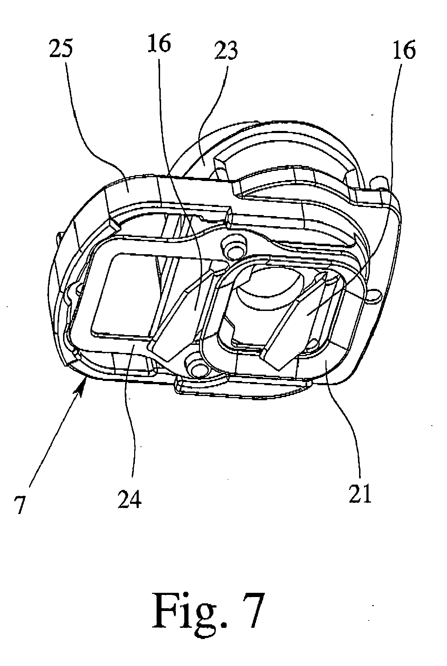

Fig. 7 shows in a perspective view the bottom of the removal device 7 according to a further embodiment.

The removal device 7 preferably forms or is formed by an insert 23 that is inserted into or connected to the mouthpiece 7.

Preferably, the opening elements 16 are at least essentially flat and/or tapered to its free ends.

Preferably, the piercing elements 16 extend at least essentially parallel to each other or away from each other (V-like).

Preferably, the piercing elements 16 are inclined to the lid 17 to be punctured, to the longitudinal extension of the carrier or blister strip 2, to the onward movement 5a, and/or to the movement of the removal device 7 for opening the respective receptacle.

Preferably, the opening elements 16 are made of metal and/or supported by a preferably flat base 24. In particular, the base 24 is also made of metal.

Preferably, the base 24 supporting or forming the opening elements 16 is connected or attached to the insert 23 and/or a supporting part 25 of the removal device 7.

The removal device 7, insert 23 and/or supporting part 25 are preferably made of plastic and/or by injection molding. It has to be noted that the opening elements 16 could also be made of plastic and/or by injecting molding although this is not preferred in the present embodiment.

The sealing 21 is preferably made of plastic and/or formed by injecting molding as well.

In particular, the sealing 21 is formed integral or in a unitary manner with the removal device 7 or the insert 23 or the supporting part 25 or the base 24 or any other suitable component.

Most preferably, the sealing 21 is directly formed by the removal device 7 or supporting part 25 or the like.

Preferably, the sealing 21 is injection molded as well, in particular bi-injected (injected into the same mould) with or onto the removal device 7, insert 23, supporting part 25 or the like.

However, the sealing 21 can also be made separately and, then, attached to the removal device 7 or any other component of the inhaler 1, preferably, by clamping, clipsing, gluing, welding, in particular ultrasound welding, or in any other suitable manner.

In the present embodiment, the sealing 21 is preferably supported or formed by or attached to the removal device 7. However, the sealing 21 could be attached also to any other component of the inhaler 1.

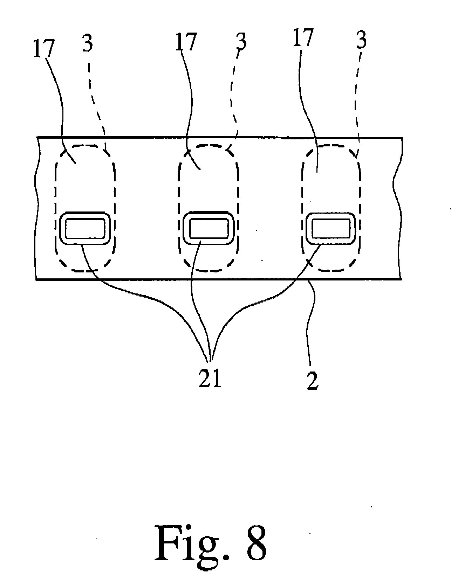

Alternatively, the sealing 21 could also be attached onto or formed by the carrier, in particular its foil, cover or lids 17 covering the blister pockets 3. This alternative is schematically shown in Fig. 8 representing a carrier, in particular a blister strip 2, with sealings 21 formed by or attached to the lids 17.

As in the previous embodiments, the sealings 21 preferably form a closed loop around the outlet opening 19 that is formed when the respective receptacle (blister pocket 3) is opened or pierced by the removal device 7,

The sealings 21 are preferably made of plastic or the like and/or directly adhered onto the foil or the like forming the cover or lids 21 of the blister strip 2.

It has to be noted with regard to all embodiments that it is also possible to arrange the sealing 21 only around the inlet opening 18 or the opening element 16 forming the inlet opening 18, However, this alternative is less preferred.

Individual features and aspects of the embodiments and the embodiments itself may be combined with one another as desired and/or used in other inhalers 1 and/or independently.

It has to be noted that Fig. 3 shows only very schematically, in particular not in scale, a potential construction. Other constructional solutions are possible as a well.

Preferably, the inhaler 1 is portable, works only mechanically and/or is handheld.

Preferably, the terms "blister strip" and "blister pockets" have to be understood in a very broad sense to cover also other kinds of storage means with receptacles or even bulk storages for the formulation.

### List of reference numbers

- 1: inhaler
- 2: blister strip
- 3: blister pocket
- 4: reservoir
- 5: conveyor
- 5a: onward movement
- 6: opening and/or removal position
- 7: piercing member
- 8: mouthpiece
- 9: air stream
- 10: powder
- 11: aerosol cloud
- 12: actuator
- 12a: housing
- 13: receiving apparatus
- 14: conveying wheel
- 15: mouthpiece cover
- 15a: cover axis
- 16: first piercing element
- 17: lid
- 18: inlet opening
- 19: outlet opening
- 20: bypass air stream
- 21: sealing
- 22: outlet path
- 23: insert
- 24: base
- 25: supporting part

## Claims

1. Inhaler (1) for delivering a preferably powdered formulation from a preferably band-shaped carrier, with a plurality of receptacles, such as blister pockets (3), containing the formulation in doses,
with a removal device (7) for separately opening the receptacles by forming an inlet opening (18) and an outlet opening (19) in the respective receptacle or a side or lid (17) of the respective receptacle, so that a gas stream, in particular an air stream (9), can flow through the inlet opening (18) into the opened receptacle and through the outlet opening (19) out of the opened receptacle to entrain and discharge the formulation contained in the opened receptacle,
**characterized in**
**that** a sealing (21) is provided or formed between the inlet opening (18) and the outlet opening (19) and/or only around the outlet opening (19) for preventing or minimizing or restricting a bypass gas/air stream (20) from the inlet opening (18) to the outlet opening (19) on the outside of the receptacle, side or lid (17).

2. Inhaler according to claim 1, **characterized in that** the sealing (21) is arranged outside the receptacles and/or carrier.

3. Inhaler according to claim 1 or 2, **characterized in that** the sealing (21) contacts the opened lid (17) from the outside and/or is pressed against the lid (17) in a removal position.

4. Inhaler according to one of the preceding claims, **characterized in that** the sealing (21) is at least partially elastically deformable, in particular comprises or formed by an elastic element.

5. Inhaler according to one of claims 1 to 3, **characterized in that** the sealing (21) is rigid.

6. Inhaler according to one of the preceding claims, **characterized in that** the sealing (21) is supported or formed by or attached to the removal device (7), and or/that the sealing (21) is injection moulded and/or unitary with the removal device (7).

7. Inhaler according to one of the preceding claims, **characterized in that** the sealing (21) forms or comprises a ring or loop-like arrangement, preferably encompassing only the outlet opening (19) and/or only an opening element (16) of the removal device (7) for forming the outlet opening (19).

8. Inhaler according to one of the preceding claims, **characterized in that** the sealing (21) forms or comprises a preferably flexible and/or elastic lip.

9. Inhaler according to one of the preceding claims, **characterized in that** the sealing (21) forms a preferably line-like contact area with the lid (17), in particular wherein the contact area is much smaller than the area of the lid (17).

10. Inhaler according to one of the preceding claims, **characterized in that** the removal device (7) comprises two opening elements (16) for forming the inlet opening (18) and the outlet opening (19), preferably wherein the opening elements (16) are constructed as cutting, tearing or piercing elements and/or the opening elements (16) are rigidly attached to a mouthpiece (8) of the inhaler (1).

11. Inhaler according to one of the preceding claims, **characterized in that** the inhaler (1) is constructed such that by breathing in while inhaling an air stream (9) of ambient air can be sucked through the inlet opening (18) into the opened receptacle and through the outlet opening (19) out of the opened receptacle for discharging the respective dose with the ambient air preferably as an aerosol cloud (11).

12. Inhaler according to one of the preceding claims, **characterized in that** the carrier is constructed in the form of a band and/or a blister strip (3), and/or that the receptacles are formed by blister pockets (3) of the carrier.

13. Carrier, preferably for an inhaler (1), with a plurality of receptacles containing a preferably powdered formulation in doses,
**characterized in**
**that** the carrier comprises sealings (21) associated to the receptacles for preventing or minimizing or restricting a bypass gas / air stream when discharging the formulation from the receptacles.

14. Carrier according to claim 13, **characterized in that** the sealings (21) are located on lids (17) or any other part of the receptacles where an outlet opening (19) is formed when discharging the formulation from the respective receptacle, in particular wherein the sealings (21) are respectively encircling that part.

15. Carrier according to claim 13 or 14, **characterized in that** the sealings (21) are flexible, rib-or lip-like and/or forming closed loops.
